# EUROPEAN PATENT SPECIFICATION

(11) **EP 0 686 136 B1**
(45) Date of publication and mention of the grant of the patent: **11.08.1999**
(21) Application number: 94910684.3
(22) Date of filing: 16.02.1994
(51) Int. Cl.: C07B 39/00, C07C 25/13, C07C 17/093, C07C 17/38

(54) **IMPROVED PROCESS FOR MANUFACTURE OF FLUOROAROMATICS**
VERFAHREN ZUR HERSTELLUNG VON FLUOROAROMATISCHEN VERBINDUNGEN
PROCEDE AMELIORE DE FABRICATION D'AROMATES FLUORES

(30) Priority: 24.02.1993 US 21541
(43) Date of publication of application: 13.12.1995
(73) Proprietor: E.I. DU PONT DE NEMOURS AND COMPANY, Wilmington Delaware 19898 (US)
(72) Inventor: KRACKOV, Mark, Harry, West Chester, PA 19382 (US); ROLSTON, Charles, Hopkins, Woodbury, NJ 08096 (US)
(74) Representative: Woodcraft, David Charles
(86) International application number: US9401478
(87) International publication number: WO9419299

(56) References cited:
- EP-A- 0 330 420
- WO-A-90/09957
- US-A- 2 939 766

## Description

### BACKGROUND OF THE INVENTION

The invention relates to a process for recovering HF from systems comprising ammonium bifluoride, hydrogen fluoride and water, and, more particularly, for recovering HF in the manufacture of fluoroaromatics by the addition of sulfur trioxide or a solution of sulfur trioxide in sulfuric acid (hereinafter referred to as "oleum") to a spent reaction mass.

U.S. Patent No. 4,912,268, discloses a process for the preparation of a fluoroaromatic compound which comprises feeding an aromatic amine in the presence of hydrogen fluoride to a reaction zone simultaneously with a diazotizing agent so as to effect diazotization of the aromatic amine, thermally decomposing the resulting diazonium salt substantially as it is formed, and removing the resulting fluoroaromatic from the reaction zone substantially as it is formed. While this method has distinct advantages over the prior art further improvements are desirable. These are centered in the work-up of the spent reaction mass from the reaction zone. The spent reaction mass, which contains substantially no fluoroaromatic product, comprises hydrogen fluoride, most of the water produced in the reaction, by-product fluorobiaryls, ammonium bifluoride and tarry non-volatile organic by-products. The water and hydrogen fluoride form an azeotrope with boiling point 111°C. When work-up of the spent reaction mass is attempted by distillation only about 65% of the hydrogen fluoride is recovered substantially free of water, by-product fluorobiaryls tend to steam distill and clog the condensers, and the still bottoms are viscous tarry non-volatile organic by-products and thus difficult to handle.

It is known in the art that sulfur trioxide or oleum can be used to recover hydrogen fluoride from its azeotrope with water. U.S. Patent 2,939,766 relates to a method for the recovery of hydrogen fluoride from a mixture of approximately one molecular proportion of alkali metal bifluoride, specifically sodium bifluoride, approximately one molecular proportion of hydrogen fluoride-water azeotrope ad up to twenty-five molecular proportions of hydrogen fluoride by adding two molecular proportions of sulfur trioxide and thereafter distilling the mixture to recover the hydrogen fluoride. U.S. Patent 5,032,371 discloses a continuous process for the recovery of anhydrous hydrogen fluoride by contacting an aqueous solution of an alkali metal fluoride in hydrogen fluoride with a sulfur trioxide-containing dehydrating stream. The process was said to be useful for the recovery of hydrogen fluoride from the alkali metal-containing heels produced in the processes for the production of aromatic fluorides. The use of sulfuric acid is said to be "advantageous as many of the possible contaminants and impurities remain dissolved in the sulfuric acid throughout the process" and because "sulfuric acid also contributes to the fluidity of the waste". There is no mention of the use of this process for the recovery of hydrogen fluoride from aqueous ammonium bifluoride solutions in these two patents.

### SUMMARY OF THE INVENTION

It has now been found that in the presence of SO₃ or oleum, HF is efficiently recovered from an aqueous waste stream which is generated in manufacturing fluoroaromatics, for example, fluorobenzene from aniline. The process allows for numerous processing advantages such as greatly reducing the build-up of fluorobiphenyls in the HF recovery system and reducing build-up of viscous tarry non-volatile organic by-products in the still bottoms. Generally, the invention relates to HF recovery from systems comprising ammonium bifluoride, hydrogen fluoride and water.

According to the present invention, there is provided a process for making a fluoroaromatic compound by diazotizing an aromatic amine in HF and decomposing the resulting aryldiazonium fluoride as it is formed wherein a liquid phase emanating from a reaction zone comprises hydrogen fluoride, water, fluorobiaryls, ammonium bifluoride and tarry non-volatile organic by-products, comprising:
(a) contacting the liquid phase emanating from the reaction zone with sufficient sulfur trioxide to convert its water content to sulfuric acid, and to destroy at least 65% of the fluorobiaryls and to convert the ammonium bifluoride to hydrogen fluoride and ammonium bisulfate, and
(b) recovering the hydrogen fluoride from the treated liquid phase with sulfur trioxide by distillation.

### DETAILED DESCRIPTION OF THE INVENTION

The present invention may be used as an improvement to the process for the manufacture of fluoroaromatics described in U.S. Patent 4,912,268, the teachings of which are incorporated herein by reference. It relates particularly to the improved processing of fluorobenzene from aniline. The improvement of this invention comprises addition of sufficient sulfur trioxide, or a solution of sulfur trioxide in sulfuric acid to a spent reaction mass, i.e. to the substantially product-free liquid from the reactor, to convert the water content of the spent reaction mass to sulfuric acid and the ammonium bifluoride content to ammonium bisulfate and hydrogen fluoride, and of recovering the hydrogen fluoride contained therein by distillation. The amount of sulfur trioxide or oleum required to complete the conversation may be readily calculated to those skilled in the art, and is further exemplified hereinafter.

More generally, the invention relates to recovery of hydrogen fluoride from systems comprising ammonium bifluoride, hydrogen fluoride, and water by the addition of sufficient sulfur trioxide or oleum to convert its ammonium bifluoride content to ammonium bisulfate and hydrogen fluoride, to convert its ammonium bifluoride content to ammonium bisulfate and hydrogen fluoride, to convert its water content to sulfuric acid, and of recovering the hydrogen fluoride contained therein by distillation.

This invention allows recovery of over 90% of the hydrogen fluoride free of water. The recovered HF may then be recycled back to the reaction zone. It is a further advantage of this invention that by-product fluorobiaryls, which otherwise steam distill and cause severe condenser plugging problems, are reduced by up to 99%, greatly diminishing the condenser clogging problem. It is yet a further advantage of this invention that the addition of sulfur trioxide or oleum alters the physical properties of tarry non-volatile organic by-products which constitute the still bottoms, converting them from a sticky residue to freely suspended solids in sulfuric acid and greatly facilitating discharge and disposal of the still bottoms.

The process of this invention may be operated on spent reaction mass either batchwise or continuously.

A preferred embodiment of this invention includes the addition of sulfur trioxide to the reactor overflow material at a lower temperature followed by a higher temperature distillation for hydrogen fluoride removal. This allows the destruction of the fluorobiphenyls at a temperature well below the temperature at which they are carried overhead by distillation. The effect of temperature on HF recovery is given in the tables below. When the sulfur trioxide addition is made at higher temperatures, a substantially reduced level of fluorobiphenyl destruction results since distillation strips the fluorobiphenyls from the sulfur trioxide-sulfuric acid medium before they can react. If the fluorobiphenyls are carried overhead, this leads ultimately to severe condenser plugging problems. While equipment plugging was not a problem on a laboratory scale because of the small amounts of materials processed, during commercial scale operations plugging from fluorobiphenyls was encountered.

The following examples and tables are to be construed as merely illustrative and not limitative of the scope of the invention.

### EXAMPLE 1

A 150 g sample of overflow material from a fluorobenzene reactor was charged to a stirred 500 ml vessel equipped with a mechanical agitator, distillation head and brine-cooled take-off condenser leading to a refrigerated receiver. The condenser was composed of silicon carbide and all other internal surfaces in the distillation and receiver were coated with Teflon® fluorocarbon resin to prevent corrosion. The composition of this material was 8.9% water, 11.6% ammonium bifluoride, 75.9% hydrogen fluoride, 0.3% mixed fluorobiphenyls, and 3-4% of both suspended and dissolved non-volatile organic by-products or tars. To the chilled mixture was slowly added 59.5 g of sulfur trioxide, the calculated amount required for complete reaction with the water in the sample. The sulfonated reaction mass was then heated; a hydrogen fluoride distilled off, the temperature slowly rose to 100°C, at which point the temperature was held constant and the distillation continued until no more hydrogen fluoride distilled. The distillate contained 119.0 g of hydrogen fluoride, 0.5 g of sulfate, and 0.7 g of water. This quantity of hydrogen fluoride corresponds to a 93% recovery of the total inorganic fluoride in the sample, including that charged a ammonium bifluoride. 97% of the fluorobiphenyls in the sample was destroyed. The non-volatile organic by-product tars in the sample were fluidized by the sulfur trioxide treatment, remaining suspended in the predominantly sulfuric acid heel at the end of the distillation.

### EXAMPLE 2

Sulfur trioxide and reactor overflow material from the fluorobenzene process, in a ratio to assure that all of the water present would be reacted, were fed simultaneously and continuously to a distillation heel such as that produced in Example 1 and at a rate such that the boiling point of the mixture was maintained at 100°C. Hydrogen fluoride was continuously taken off overhead with the extent of recovery and composition similar to that obtained in Example 1. From 150 g of overflow material fed in this manner (at a rate of 2.0 g/min), 118.2 g of hydrogen fluoride containing 0.8% sulfate and 0.4% water was collected. The inorganic fluoride recovery a hydrogen fluoride was 92%. About 65% of the fluorobiphenyls were destroyed.

As in Example 1, the non-volatile organic by-products were converted from a sticky residue that tended to adhere to vessel walls to freely suspended solids in the predominantly sulfuric acid heel was produced during the distillation.

### EXAMPLE 2A

Sulfur trioxide was fed continuously to a recirculating stream of reactor overflow material from the fluorobenzene process held at 50°C in a vessel with a residence time of at least 3 hours, in a ratio to insure that all of the water present would be reacted, and to which reactor overflow material was continuously fed. The material treated with sulfur trioxide was withdrawn from this vessel at a rate equivalent to the overflow feed rate and fed to a distillation heel such as that produced in Example 1, maintained at a boiling point of 100°C. Hydrogen fluoride was continuously taken off overhead with the extent of recovery and composition similar to that obtained in Example 1. From 150 g of overflow material fed in this fashion, 118.0 g of hydrogen fluoride containing 0.8% sulfate and 0.4% water was collected; recovery of hydrogen fluoride was 92%. 90% of the fluorobiphenyls were destroyed.

As in Example 1, the non-volatile organic by-products were converted from a sticky residue that tended to adhere to the vessel walls to freely suspended solids in the predominantly sulfuric acid heel that was produced in the distillation.

### COMPARATIVE EXAMPLE

The apparatus, procedure, and feed was as described in Example 1, except that sulfur trioxide was not added to the reactor overflow material before distiling the hydrogen fluoride from it. Recovery of hydrogen fluoride was 64% under these circumstances; no significant reduction of the fluorobiphenyls initially present was observed. In the absence of added sulfur trioxide, the non-volatile organic by-product tar residues coated the internal Teflon® fluorocarbon resin-coated surfaces of the distillation vessel with a sticky black coating.

### TABLES

The following tables show the effect of temperature on HF recovery. Although the recovery of HF is maximized at temperatures of 140°C or above, it will be appreciated by those skilled in the art that process operability factors may dictate operating below the temperature for maximum recovery.

### HF Recovery-Effect of S0₃ Treatment Distillation of Reactor Overflow

The composition comprises 67.5% HF, 13.5% NH₄HF₂ and 13.4% H₂O, 3.6% TARS/DOC. The total HF content (HF + NH₄HF₂) was 77.3g/100g O/F; SO₃ requirement (1:1 SO₃ - H₂O) was 59.5g/100g O/F; and 65% oleum requirement (1:1 SO₃ - H₂O) was 91.7g/100g O/F. The following table shows HF recovery versus distillation temperature for SO₃ using stoichiometric 1:1 SO₃-H₂O for all distillations.

| Temperature °C | Reactor O/F, g | Distillation HF, g | Total HF Recovery, % |
|---|---|---|---|
| 80 | 100 | 62.6 | 81.0 |
| 90 | 100 | 63.8 | 82.5 |
| 100 | 100 | 68.5 | 88.6 |
| 110 | 100 | 70.2 | 90.8 |
| 120 | 100 | 71.5 | 92.5 |

The following table shows HF recovery versus distillation temperature for 65% oleum using stoichiometric 1:1 S03-H20 for all distillations.

| Temperature °C | Reactor O/F, g | Distillation HF, g | Total HF Recovery, % |
|---|---|---|---|
| 80 | 100 | 54.7 | 70.8 |
| 90 | 100 | 64.7, 60.8 | 83.7, 78.7 |
| 100 | 100 | 67.4 | 87.2 |
| 110 | 100 | 67.8 | 87.7 |
| 120 | 100 | 72.0 | 93.2 |
| 130 | 100 | 71.6 | 92.6 |
| 140 | 100 | 72.2, 73.2 | 93.4, 94.8 |

The following comparative table shows the HF recovery versus distillation temperature in the absence of S0₃ or oleum.

| Temperature °C | Reactor O/F, g | Distillation HF, g | Total HF Recovery, % |
|---|---|---|---|
| 90 | 100 | 35.3 | 45.7 |
| 100 | 100 | 40.3 | 52.1 |
| 110 | 100 | 45.9 | 59.4 |

Comparable results have beer, demonstrated using synthetic mixtures of a composition comprising 78.8% HF, 9.2% H₂O, 12.0% NH₄HF₂. The total HF content (HF + NH₄HF₂) was 87.2g/100g O/F; SO₃ requirement (1:1 SO₃ - H₂O) was 40.8g/100g O/F; and 65% oleum requirement (1:1 SO₃ - H₂O) was 62.8g/100g O/F. That is, by adding a amount of SO₃ or oleum equivalent to the water content of the mass, significant enhancement of HF recovery can be obtained.

## Claims

1. A process for making a fluoroaromatic compound by diazotizing an aromatic amine in HF and decomposing the resulting aryldiazonium fluoride as it is formed wherein a liquid phase emanating from a reaction zone comprises hydrogen fluoride, water, fluorobiaryls, ammonium bifluoride and tarry non-volatile organic by-products, comprising:
(a) contacting the liquid phase emanating from the reaction zone with sufficient sulfur trioxide to convert its water content to sulfuric acid, and to destroy at least 65% of the fluorobiaryls and to convert the ammonium bifluoride to hydrogen fluoride and ammonium bisulfate, and
(b) recovering the hydrogen fluoride from the treated liquid phase with sulfur trioxide by distillation.

2. A process according to claim 1, wherein the sulfur trioxide is a solution of sulfur trioxide in sulfuric acid.

3. A process according to claim 1 to 2, wherein the fluoroaromatic compound is fluorobenzene and the aromatic amine is aniline.

## Patentansprüche

1. Verfahren zur Herstellung einer fluoraromatischen Verbindung durch Diazotierung eines aromatischen Amins in HF und Zersetzung des resultierenden Aryldiazoniumfluorids unmittelbar nach seiner Bildung, in welchem eine flüssige Phase, die aus einer Reaktionszone hervorgeht, Fluorwasserstoff, Wasser, Fluorbiaryle, Ammoniumbifluorid und teerartige nichtflüchtige organische Nebenprodukte umfaßt, umfassend:
(a) Kontaktieren der flüssigen Phase, die aus der Reaktionszone hervorgeht, mit ausreichend Schwefeltrioxid, um deren Wassergehalt in Schwefelsäure umzuwandeln und mindestens 65% der Fluorbiaryle zu zerstören und das Ammoniumbifluorid in Fluorwasserstoff und Ammoniumbisulfat umzuwandeln, und
(b) Gewinnung des Fluorwasserstoffs aus der behandelten flüssigen Phase mit Schwefeltrioxid durch Destillation.

2. Verfahren nach Anspruch 1, in welchem es sich bei dem Schwefeltrioxid um eine Lösung von Schwefeltrioxid in Schwefelsäure handelt.

3. Verfahren nach Anspruch 1 oder 2, in welchem die fluoraromatische Verbindung Fluorbenzol ist und das aromatische Amin Anilin ist.

## Revendications

1. Procédé de fabrication d'un composé aromatique fluoré par diazotation d'une amine aromatique dans du HF et décomposition du fluorure de diazonium d'aryle obtenu a mesure qu'il est formé, dans lequel une phase liquide émanant d'une zone de réaction comprend du fluorure d'hydrogène, de l'eau, des fluorobiaryles, du difluorure d'ammonium et des produits secondaires organiques non-volatils goudronneux, comprenant :
(a) la mise en contact de la phase liquide émanant de la zone de réaction avec suffisamment de trioxyde de soufre pour convertir sa teneur en eau en acide sulfurique, et pour détruire au moins 65% des fluorobiaryles et pour convertir le difluorure d'ammonium en fluorure d'hydrogène et en disulfate d'ammonium, et
(b) la récupération de fluorure d'hydrogène à partir de la phase liquide traitée avec le trioxyde de soufre par distillation.

2. Procédé suivant la revendication 1, dans lequel le trioxyde de soufre est une solution de trioxyde de soufre dans de l'acide sulfurique.

3. Procédé suivant les revendications 1 à 2, dans lequel le composé aromatique fluoré est le fluorobenzène et l'amine aromatique est l'aniline.
